# EUROPEAN PATENT SPECIFICATION

(11) **EP 0 462 620 B1**
(45) Date of publication and mention of the grant of the patent: **26.07.1995**
(21) Application number: 91110249.9
(22) Date of filing: 21.06.1991
(51) Int. Cl.: D04H 1/54

(54) **Process for bonding blends of cellulosic pulp and fusible polyolefin or polyester pulp by high-speed dielectric heating and products produced thereby**
Verfahren zum Verbinden von Mischungen aus Cellulosepulpe und schmelzbarer Polyolefin- oder Polyesterpulpe durch dielektrische Hochgeschwindigkeitsheizung und dadurch erhaltene Produkte
Procédé pour lier des mélanges de pâte cellulosique et pâte polyoléfinique ou polyester fusibles par chauffage diélectrique de grande vitesse et produits obtenus

(30) Priority: 21.06.1990 US 541569
(43) Date of publication of application: 27.12.1991
(73) Proprietor: E.I. DU PONT DE NEMOURS AND COMPANY, Wilmington Delaware 19898 (US)
(72) Inventor: Williams, Kenneth Roger, Landenberg, Pennsylvania 19350 (US); Fitzgerald, Cornelius Gilbert, St. Petersburg, Florida 33713 (US)
(74) Representative: Abitz, Walter, Dr.-Ing.

(56) References cited:
- GB-A- 1 092 373
- US-A- 3 287 474

## Description

### FIELD OF THE INVENTION

The invention relates to a process for bonding blends of dry cellulosic fluff pulp and fusible polyolefin or polyester pulp or fiber by high-speed dielectric heating in the absence of any dielectric sensitizing material. The invention also relates to products produced by the bonding process.

### BACKGROUND OF THE INVENTION

The use of disposable hygienic products, such as baby diapers, adult incontinence products and feminine napkins, is widespread in developed nations. All of these products are designed to absorb body fluids efficiently and at low cost. Typically, the principal absorbent material used in the core of these products is cellulosic fluff pulp. The cellulosic fluff pulp is usually obtained by defibering a pulp sheet in a hammermill or a pin mill. Although cellulosic fluff pulp is relatively inexpensive, its high weight percentage in the final product makes it the major material cost of the hygienic disposable product. Additionally, increasing environmental concerns dictate that disposable products be lower in volume and weight so that they don't take up as much landfill space. As a result, there is an increasing need to reduce the quantity of cellulosic fluff pulp in disposable products.

One technique that has found widespread acceptance in the baby diaper industry is to incorporate one or more super absorbent polymers (hereinafter "SAP") into the cellulosic fluff pulp. The absorption capacity index (i.e., g fluid absorbed/ g of absorbent) for diaper cores containing SAP is significantly greater than that for cores containing no SAP, so the total core weight of the disposable product can be reduced while still maintaining equivalent total absorption.

Another technique for reducing the quantity of cellulosic fluff pulp in the final disposable product is to thermally bond the cellulosic fluff pulp with a small amount (e.g., 2-30 wt. %) of a fusible synthetic pulp or fiber that is intermixed therewith. The resulting bonded pulp has greatly increased tensile and compressive strength over unbonded pulps. In addition, fluid absorption, particularly under load, is also increased. As a consequence of such bonding, the core weight of the disposable product can be reduced significantly, while still maintaining equivalent total absorption.

In such bonding applications, a thermal bonding device is necessary to realize reductions in core weight. Since the fluff pulp core is created during the manufacturing process, it is highly desirable for the thermal bonding device to be an integral part of the process. Although it is possible to make bonded cores separately, and then to combine them on the production line with other elements of the disposable product, such steps are economically unrealistic. Several specific bonding techniques have been proposed, but there are significant speed and safety constraints that have sharply limited commercialization of these techniques.

The presently preferred bonding technique practiced by the disposable art utilizes a "through air" system in which air, heated above the fusion temperature of the fusible synthetic pulp or fiber, is introduced into a substantially closed container. The intermixed pulp material travels around a perforated drum or on a mesh belt which is under vacuum, so that heated air is drawn through the pulp material thereby heating it above the fusion temperature of the synthetic pulp or fiber. The bonding step is critical because, at the operating speeds of present day production lines, fusion must be accomplished in a fraction of a minute, ideally two seconds or less. Fusion time can be increased by increasing the length of travel in the thermal bonder, but this approach leads to excessively large and costly bonding units.
Another alternative is to use radiant energy to bond the pulp material. However, radiant heating creates a significant safety hazard because the equipment utilized operates at temperatures above the ignition point of cellulose.

Still another technique is to use dielectric heating, but there are formidable problems with the use of this type of technology. Dielectric heating involves rapid and uniform heating throughout a nonconducting material by means of a high-frequency electromagnetic field. Commonly, this includes radio frequency (hereinafter "RF") and microwave energy. Most commercially available RF or microwave heaters are large and the product moves through them slowly. Power can be increased, but there is a limitation in this application since no arcing can be tolerated due to the extreme fire danger produced by pulp fines floating in the atmosphere. Therefore, they are not suited for an in-line thermal bonding operation useful for manufacturing hygienic disposable products.

U.S. Patent No. 3,287,474, for example, uses heat which is generated from electrical sparks to fuse heat fusible binder material for preparing non-woven fabrics. This method is not suited for the present purposes.

Finally, the fusible synthetic pulp or fibers (e.g., polyolefins and polyesters) have low dielectric loss factors which make them unreactive at radio frequencies. Existing commercially-marketed synthetic fibers in general, and polyester fibers in particular, do not heat up in an oscillating electromagnetic field and cannot be thermally bonded. Indeed, polyesters are good insulators and have low dielectric or inductive loss. This property is why polyesters are useful in capacitors.

British Patent No. 1,092,373, for example, describes a method for making non-woven fabrics of the bonded-web type by using dielectric heating for the bonding step, however, it is necessary that the fibers comprise at least 5% fibers which exhibit a dielectric loss.

In order to overcome the above-noted problem, the costly step of incorporating a polar material in the manufacture of the fusible synthetic pulp or fiber has been considered necessary to achieve rapid dielectric heating to fusion temperatures. For example, the approach adopted in U.S. Patent No. 4,401,708, directed to a method of bonding nonwoven fabrics using microwave energy and a polar trichloroacetic acid solvent, presents significant control problems in applying the solvent to appropriate pulp or fiber locations without excessive degradation of the pulp or fiber upon prolonged exposure to the solvent. Control problems lead to nonuniformly bonded products.

Although techniques for thermally bonding absorbent fluff pulp cores have been known for many years, their adoption by the hygienic disposable product industry has been very slow. Reasons for this situation include the unsatisfactory size, cost and safety of commercially available bonding units.

As a result, what is needed is a process to improve the speed of thermal bonding over what has previously been possible commercially. Another objective is to overcome some of the limitations that have been apparent, and even inherent, in various prior commercial bonding techniques. Moreover, cost-effectiveness is always an important objective for any commercial operation.

Other objects and advantages of the present invention will become apparent to those skilled in the art upon reference to the attached drawings and to the detailed description which hereinafter follows.

### SUMMARY OF INVENTION

In accordance with the present invention, there is provided a process for bonding blends of cellulosic fluff pulp and fusible low melting polyolefin or polyester pulp or fiber by high-speed dielectric heating. The process comprises, as a first step, blending a dry cellulosic fluff pulp with a polyolefin or polyester pulp or fiber having a melting point less than 150°C, such that the resulting pulp blend has a density of between about 0.02 and 0.50 g/cc and comprises between 70-98 parts cellulosic fluff pulp and between 2-30 parts polyolefin or polyester pulp or fiber. Thereafter, the blended pulp is bonded in less than 15 seconds by dielectric heating. Bonding occurs at temperatures which are apparently below the melting point of the polyolefin or polyester pulp or fiber when instrumentally measured inside the blended pulp.

The invention recognizes that polyolefin or polyester pulps and fibers, preferably polyethylene, having a melting point less than 150°C, which are normally unreactive to high frequency electromagnetic fields like radio frequencies and microwave energy, can be bonded in the absence of dielectric sensitizing materials when intimately mixed with a dry cellulosic fluff pulp.

In another aspect of the invention, there is provided a dielectrically bonded article having a density between about 0.02 and about 0.50 g/cc, comprising 70-98 parts of a cellulosic fluff pulp and 2-30 parts of a fusible polyolefin or polyester pulp or fiber having a melting point less than 150°C. The tensile strength of the interior of the bonded article is at least 120% greater than the tensile strength of any surface portion of the bonded article. The bonded article of the invention is particularly useful as an absorptive core in disposable hygienic products, preferably baby diapers, adult incontinence products or feminine pads. In addition, the bonded article may also be used as furniture padding, cushioning material or polishing pads.

As used herein, "high-speed dielectric heating" means that the blended cellulosic fluff pulp and fusible polyolefin pulp can be bonded by a high frequency electromagnetic field in less than 15 seconds, preferably within 1 to 2 seconds. Non-limiting examples that can produce the desired high frequency electromagnetic field include radio frequencies ("RF") and microwave energy.

As used herein, "dry" means that the cellulosic fluff pulp has less than 6 wt.% moisture but contains "bound water" as defined in Cellulose and Cellulose Derivatives, Part I, by E. Ott and H. Spurlin, Editors, High Polymer Series, Vol. 5, Interscience, pp. 400-402 (1954).

### BRIEF DESCRIPTION OF THE DRAWINGS

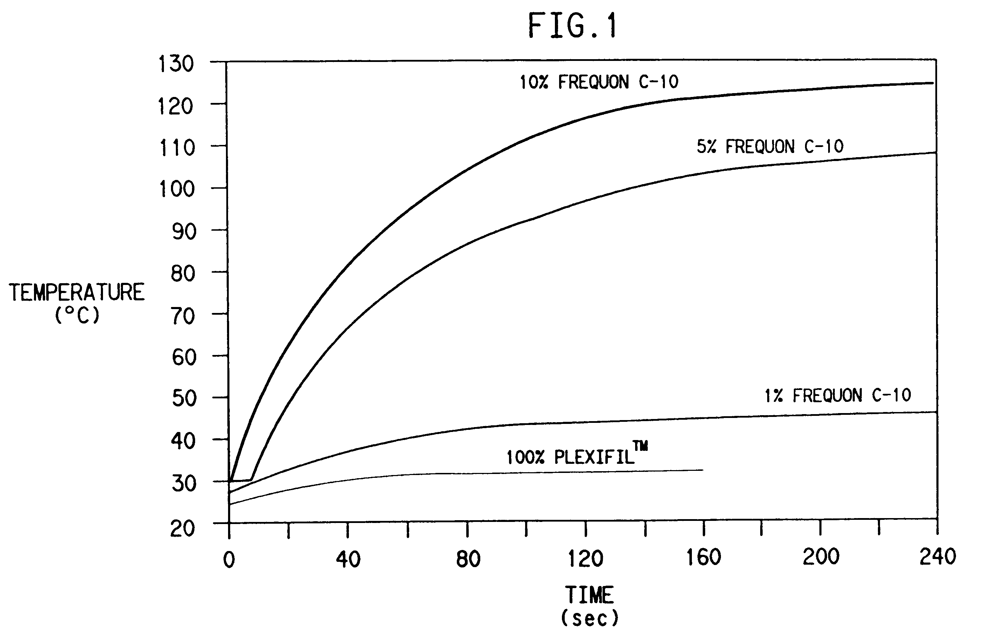

Figures 1-7 show curves plotting temperature against time for various fiber blends as described hereinafter.

### DETAILED DESCRIPTION OF PREFERRED EMBODIMENTS

In accordance with the invention, an intimately blended pulp is prepared by feeding a refined polyethylene pulp, or other low melting polyolefin pulp or fiber, in sheet form into a hammermill along with one or more dry cellulosic pulp sheets. These sheets are typically in roll form. The hammermill produces an intimately blended polyethylene and cellulosic fluff pulp. The pulps are blended so that the hammermill yields a fluff blend containing 2-30 parts polyethylene pulp and 70-98 parts cellulosic fluff pulp. The resulting pulp blend is conducted to a vacuum former where a low density (0.02-0.50 g/cc) fluff pulp web is built up on a continously moving screen.

Preferably, the cellulosic pulp sheet comprises wood pulp or cotton linters. A suitable wood pulp is commercially available from Weyerhaeuser Co. as New Bern (NB) 316 cellulose pulp. A suitable polyolefin pulp is commercially available from E. I. du Pont de Nemours & Co. under the trademark Plexafil™. It should be noted that fusible polyester pulps or fibers may be substituted for polyolefin for purposes of the invention.

Alternatively, the cellulosic and polyethylene pulp blend may contain from 0 to 30 parts of a polar material, such as a super absorbant polymer (SAP). Additionally, other adjuvants, such as odor control materials, may be added to the blended pulp to meet customer needs. However, it is to be understood that these materials are not critical to the invention since dielectric bonding will occur to the same extent with or without them.

The resulting continuous web is transferred to an insulative belt which is radio frequency insensitive and passed through a radio frequency applicator cavity. The applicator cavity as used herein refers to a tranverse electric cavity or TE101 mode microwave cavity. The TE101 mode cavity is referenced in Ramo, Whinnery and Duzer, "Fields and Waves in Communications Electronics", John Wiley, 2nd edition, page 490 (1984) and further described in Marcuvitz, "The Waveguide Handbook", MIT Radiation Labaratory Series, Volume 10 (1951). The microwave energy and applicator cavity design are adjusted so that bonding occurs in the cavity in less than 15 seconds. Preferably, bonding occurs within 1 to 2 seconds after application of the microwave energy.

It is well known that materials with a high dielectric loss factor (i.e., polar materials) will heat up in a high frequency electromagnetic field. Further, it is known that non-polar materials, such as polyolefins and polyesters, can be heated by conduction by applying dielectric sensitizing polymers which are polar to the surface or within the body of the non-polar material. Such sensitizing materials are commercially available for both RF (less than 300 MHz) and microwave frequency (greater than 300 MHz) applications.

The invention will be further described by reference to the following non-limiting examples. As used herein, all percentages are by weight unless otherwise indicated.

### EXAMPLE 1

A dielectric sensitizing polymer, commercially available from Structol Co. under the tradename Frequon C-10, was applied to numerous sheets of Plexafil™ polyolefin pulp so as to give add-on levels of 1%, 5% and 10% Frequon C-10. Plexafil™ is a trademark for a commercially available polyolefin pulp from E. I. du Pont de Nemours & Co., Wilmington, Delaware. The pulp was defibered in a laboratory blender for 1 minute to yield a low density, 0.02 g/cc fluff. The fluff pulp samples were subsequently placed in a W.T. LaRose model CCH-8.5 dielectric heater operating in a RF range of 90-91 MHz. A nonintrusive probe made by Luxtron Corporation (LUXTRON Model 755 Fluoroptic Thermometer) was inserted into the fluff structure and the temperature was measured as a function of time. These measurements are plotted in Figure 1.

Figure 1 shows that the temperature of the polyolefin pulp with no Frequon C-10 present (i.e., 100% Plexafil™) remains almost unchanged over 160 seconds. However, applying 1%, 5% and 10% concentrations of Frequon C-10 to the surface causes a continous rise in temperature up to 240 seconds. The rate of temperature rise increases with increasing Frequon C-10 concentration. The Frequon compounds exhibit high losses in an RF (radio frequency) field due do their high dielectric constants. They are manufactured by the Structol Company of Stowe, Ohio, U.S.A.

### EXAMPLE 2

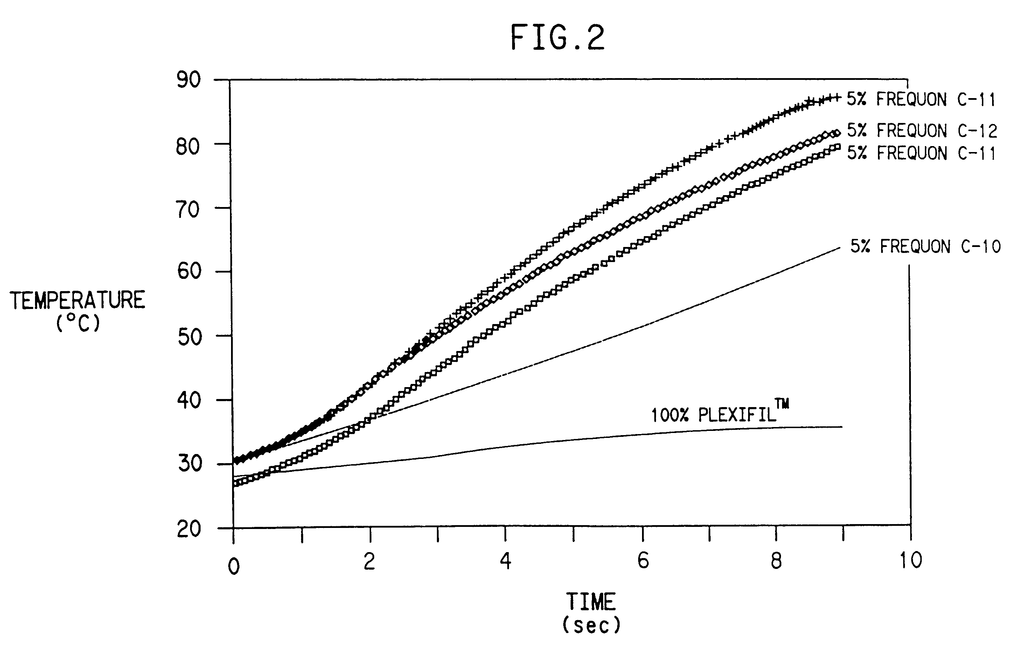

This example mirrors Example 1, except that the temperature versus time behavior was observed using microwave frequencies. The microwave unit consisted of a rectangular cavity resonator operating in a TE101 mode where the electric field is uniform across the transverse direction and has one standing wave condition (peak) in the machine and height directions. The unit was supplied with a power generator operating at a frequency of 925 MHz and approximately 400 watts. The presence of various organic polymer activators at 5% concentration, Frequon C-10, C-11, and C-12 (all commercially available through Structol Co.), caused a significant rise in temperature, while temperature was only modestly raised when no Frequons were present. These experiments are set forth in Figure 2 and show that pure polyolefin pulp in fluff form (i.e., 100% Plexafil™) cannot be heated significantly in the absence of high dielectric loss materials, like Frequon C-10, C-11 or C-12.

### EXAMPLE 3

In this example, blends of cellulose and fusible pulps or fibers were defibered by processing them in a laboratory blender. Thereafter, the blended pulp was vacuum formed into a low, approximately 0.02 g/cc, density web on a screened 7.5 cm diameter cavity. The resulting web was compressed by a plate placed on top of the blended pulp to a desired final density of about 0.09 to 0.12 g/cc prior to bonding in a dielectric heater.

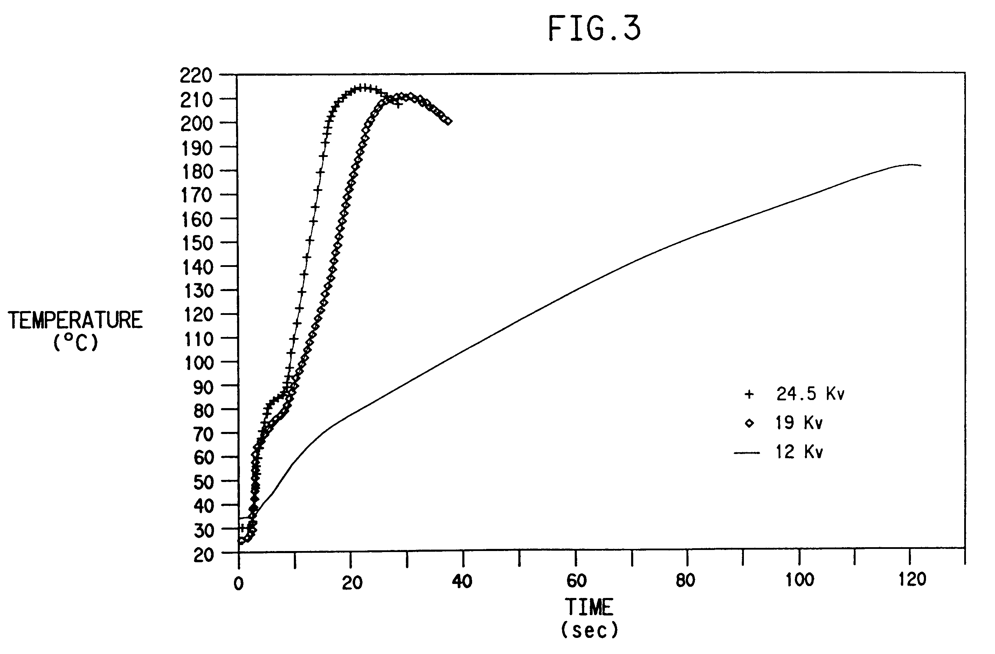

Referring now to Figure 3, the temperature rise associated with Example 3 is shown for a nonwoven fluff composed of 85 parts Weyerhaeuser NB 316 lb. kraft cellulose pulp and 15 parts Plexafil™ which contained 1% Frequon C-10 on its surface. At 12 KV, in the same RF unit as was used for Experiment 1, the temperature rose in 60 seconds to 130°C compared to 40°C in Figure 1. Higher operating voltages, 19 and 24.5 KV respectively, showed further rapid increases in recorded temperature in the blended pulp. The blend of cellulose fluff and Plexafil™ with 1% Frequon C-10 showed a more rapid temperature rise than a fluff of Plexafil™ alone with 1% Frequon C-10 (Example 1).

### EXAMPLE 4

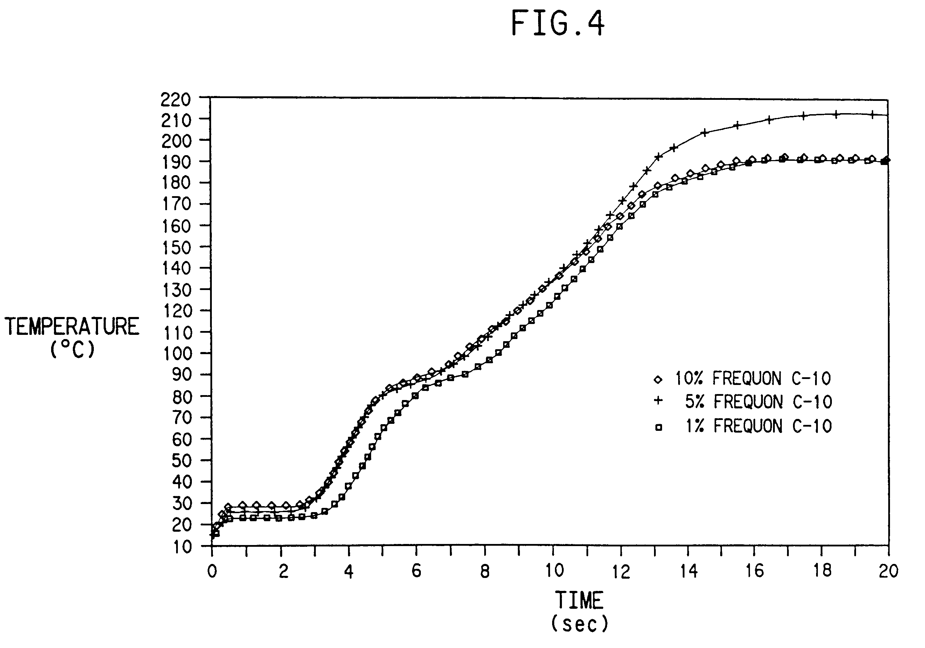

In this example, varying amounts of Frequon C-10 were applied to Plexafil™ sheets as described in Example 1. Treated Plexafil™ was processed with Weyerhaeuser NB 316 cellulose pulp as described in Example 3 and then subjected to RF radiation in a W.T. LaRose C220/100 dielectric heater at 15 KV peak voltage and 77.5 MHz frequency. Parallel plate electrodes were used. Figure 4 shows similar temperature profiles for a 0.08 g/cc fluff blend of 85 parts Weyerhaeuser pulp and 15 parts Plexafil™ in which the Plexafil™ contained varying amounts of Frequon C-10, in 1%, 5% and 10% concentrations on its surface. This is to be contrasted with the large temperature differences when Frequon concentration was varied on 100% Plexafil™ fluff shown in Figure 1.

### EXAMPLE 5

In this example, fluff composites were made, as in Example 2, of kraft pulp (Weyerhaeuser NB 316)/Plexafil™ pulp, 85/15 and 0.060-0.075 g/cc density, with and without Frequon C-10 on the surface of the Plexafil™. The composites were dielectrically heated in a W.T. LaRose C220/100 RF unit at 15 KV peak voltage and 77.5 MHz frequency. Table 1 shows probe temperature as a function of time for samples A, E, F, and J both during RF treatment and after the RF unit was turned off.

**TABLE 1**

| | Sample A | Sample E | Sample F | Sample J |
|---|---|---|---|---|
| Frequon Conc. on Plexafil™ (%) | 0 | 5 | 0 | 5 |
| Radio Frequency Application Time (sec.) | 2 | 2 | 4 | 4 |

| Time From Start of R.F. Application (sec.) | Probe Temp.(°C) | | | |
|---|---|---|---|---|
| 0 | 25.7 | 26.8 | 27.7 | 25.3 |
| 1 | 45.9 | 56.4 | 66.8 | 37.7 |
| 2 | 79.8 | 86.8 | 99.8 | 78.8 |
| 3 | 77.2 | 81.5 | 100.6 | 94.9 |
| 4 | 73.1 | 78.9 | 117.2 | 107.4 |
| 5 | 72.5 | 79.6 | 122.3 | 117.0 |
| 6 | 73.7 | 80.9 | 129.5 | 126.1 |
| 7 | 79.1 | 81.6 | 134.1 | 132.6 |
| 8 | | 82.1 | 137.3 | 136.5 |
| 9 | | 82.1 | 138.9 | 138.7 |
| 10 | | 81.7 | 140.1 | 140.8 |
| 11 | | | 140.8 | 141.5 |

The data show only modest differences in temperature rise between samples with and without Frequon C-10 on the Plexafil™. Temperature rise is controlled by the length of time RF energy is applied to the samples. Subjective determination of bonding showed that all samples exhibited some degree of bonding despite the low recorded temperature for samples A and E that never reached the fusion temperature of linear polyethylene. Samples F and J showed subjectively good bonding although the linear polyethylene fusion temperature was just reached for a few seconds. With a two second cycle time, the maximum temperature recorded occured at the end of the RF treatment period. With a four second RF cycle time, the temperature continued to rise for several seconds after the RF unit was turned off. When similar density samples were heated by convection in an oven, there was only modest bonding even after 5 minutes of treatment. This example shows that temperature rise and bonding of a cellulose fluff/fusible polyolefin pulp blend occurs to the same extent with or without the presence of a high dielectric loss material on the fusible polyolefin pulp surface.

### EXAMPLE 6

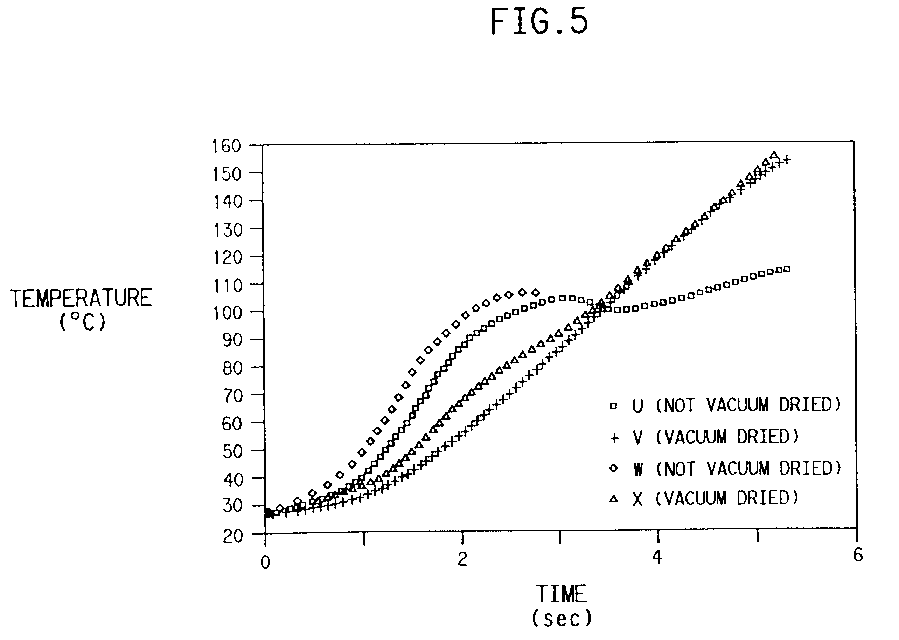

In this example, samples U, V, W and X were prepared by the same procedure as in Example 3. Samples U and V were made of 90 parts cellulose fluff, 10 parts Plexafil™ and 10 parts Stockhausen W40158 SAP. Samples W and X were made of 90 parts cellulose fluff and 10 parts Plexafil™. The samples had densities ranging from 0.06 to 0.025 g/cc and were subjected to an 8 second RF cycle time. Figure 5 shows that there is a difference in the temperature versus time curves for samples that were vacuum dried (V and X) prior to treatment, and those that were not vacuum dried (U and W). The vacuum dried samples show a reasonably constant rate of temperature rise over much of their cycle time, reaching a probe temperature well above the fusion temperature of polyethylene before the power was turned off. Under these conditions, sample V ignited. Therefore, safety considerations require that the input RF energy be adjusted so that probe temperature does not exceed 150°C while RF energy is being applied.

### EXAMPLE 7

In this example, air-laid felts were prepared, as in Example 3, of 90 parts cellulose fluff pulp and 10 parts Plexafil™, in a range of densities from 0.06 to 0.8 g/cc. All fluff systems were defibered in a laboratory blender. The samples of highest density, 0.8 g/cc, were obtained by wetting out the samples with water to make them more readily compressible. After compression, these samples were dried before testing. Table 2 illustrates the densities of the various sample numbers.

**TABLE 2**

| Sample No.* | Density(g/cc) | Sensitizer on Plexafil™(%) |
|---|---|---|
| M | 0.06 | None |
| N | 0.10 | None |
| O | 0.20 | None |
| P | 0.50 | None |
| T | 0.80 | None |
| S | 0.80 | 5% Frequon C-10 |

| | | |
|---|---|---|
| * All samples were defibered blends of 5.0 ± 0.15 g of 90 parts kraft cellulose pulp and 10 parts of Plexafil™. | | |

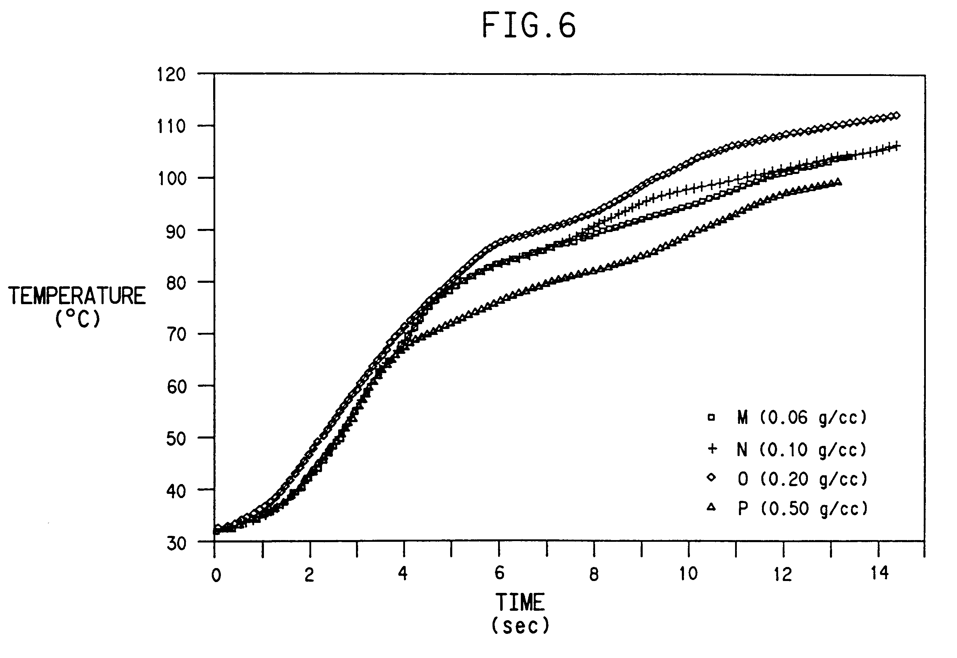

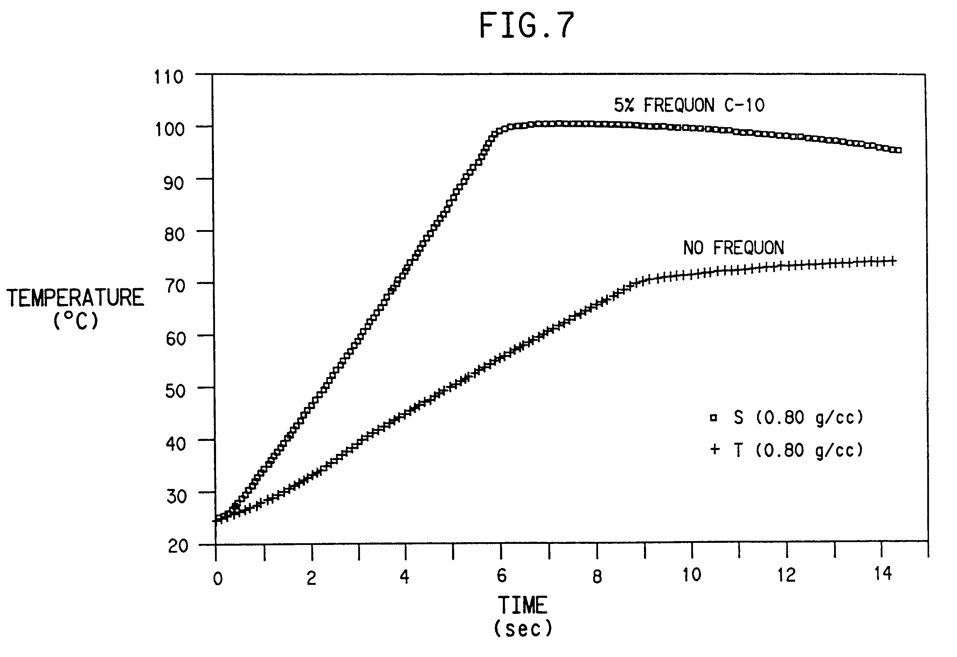

All samples were tested in a microwave applicator for 8 seconds at 922 MHz frequency and 370 watts. Figure 6 shows similar probe temperature versus time curves for all samples up to a density of 0.5 g/cc (samples M,N,O and P). Samples of density greater than 0.5 g/cc (samples T and S), shown in Figure 7, have a different temperature versus time profile. With these higher density samples, there is a sharp change in the slope of the probe temperature versus time curve. Further, sample S, which contained a Frequon C-10 dielectric sensitizer, showed a more rapid initial temperature rise. This is to be contrasted to the temperature versus time measurements made with lower density composite structures in which the presence or absence of Frequons on the fusible polyolefin pulp blend had little effect. Thus, sample density influences the time versus temperature relationship in microwave heating. As a result, cellulose fluff pulp and fusible polyolefin pulp nonwoven blends of densities below 0.50 g/cc can be dielectrically bonded more rapidly than heavier density blended pulps and they are therefore more desirable.

### EXAMPLE 8

In this example, samples were made, as in Example 3, at about 0.08 g/cc density with the following formulations:

| Sample | Cellulose (Parts) | Fusible Pulp/Fiber (Parts) | SAP* (Parts) |
|---|---|---|---|
| A | 90 | Plexafil™-10 | 0 |
| B | 96 | Plexafil™-4 | 10 |
| C | 90 | Pulpex® E-338**-10 | 0 |
| D | 90 | Dacron® Type 271***-10 | 0 |

| | | | |
|---|---|---|---|
| * Available from Stockhausen Co. as SAP W40185 | | | |
| ** A polyolefin pulp commercially available from Hercules, Inc., Wilmington, Delaware. | | | |
| *** A sheath/core, bicomponent polyester fiber available from E. I. du Pont de Nemours & Co., Wilmington, Delaware. The copolyester in the sheath has a melting point of 110°C. | | | |

All samples were treated in a W.T. LaRose C220/100 RF heater at 15 KV peak voltage and 77.5 MHz frequency. All samples showed good bonding after treatment.

### EXAMPLE 9

In this example, air-laid webs were produced in 7.5 cm diameter disk form from 85 parts Weyerhaeuser NB 316 pulp and 15 parts of Plexafil™ pulp. The disks were then thermally bonded at a density of 0.08 g/cc in two ways. The two ways included (1) heating the disks in a laboratory press at fixed caliper with the upper platten at 154°C and the lower platten at 150°C and (2) heating the disks in an RF radiation unit as described in Example 4.

Softness and hand of the disks bonded by the two techniques were readily distinguishable. The dielectrically-bonded disks had soft surfaces and firm interiors, while the press-bonded disks showed the opposite characteristics. In fact, the press-bonded disks showed the greatest degree of bonding on the surfaces. This subjective difference can also be characterized quantitatively by measuring the tensile properties of the bonded web at the surface and in the interior of the disk. The following procedure was used:

After the disks were thermally bonded, they were cut into three 7.5 cm diameter wafers of approximately equal thickness and equal weight. One wafer was cut from the middle zone of the original disk while each of the other two wafers contained one of the outer surfaces of the original disk. In other words, there were two wafers cut from the outside and one wafer from the inside of the original disk. These wafers were then tensile tested at 20°C and 60% relative humidity using an Instron unit with a 3.75 cm jaw spacing and a 1.25 cm/min. rate of extension. The sides of the wafers were trimmed so that rectangular specimens of dimensions 5 cm x 3.75 cm were tested. The breaking load and weight of the disks were determined. The ratio of the strength of the inside wafer to the strength of the outside wafer (all normalized to equivalent wafer weight) was calculated. Table 3 illustrates the differences between the press bonded disks and the RF bonded disks.

**TABLE 3**

| Strength Ratio (Inside Wafer/Outside Wafer) | | |
|---|---|---|
| Press Bonded | RF Bonded | Bonding Time(sec.) |
| 0.53 | 2.12 | 8 |
| 0.84 | 1.46 | 8 |
| 0.88 | 1.29 | 8 |
| 0.45 | | |
| | 1.12 | 4 |
| | 1.85 | 4 |
| Avg. 0.68 | 1.57 | |

The data show that RF bonded specimens are characterized by greater thermal bonding in the interior, while bonding by conduction in a press causes the surface to be more thoroughly bonded. In fact, the bonded specimens produced by the inventive process have an interior tensile strength that is at least 120% of the tensile strength of any outer surface of the bonded specimens. The soft outer surfaces of the dielectrically bonded specimens make them particularly preferred as the absorptive core in hygienic disposable products such as baby diapers.

Although particular embodiments of the present invention have been set forth in the foregoing description, it will be understood by those skilled in the art that the invention is capable of numerous modifications, substitutions and rearrangements without departing from the spirit or essential attributes of the invention. Reference should be made to the appended claims, rather than to the foregoing specification, as indicating the scope of the present invention.

## Claims

1. A process for bonding a blend of cellulosic fluff pulp and fusible polyolefin or polyester pulp or fiber comprising the steps of intimately blending a dry cellulosic fluff pulp with a polyolefin or polyester pulp or fiber having a melting point less than 150°C, such that the resulting blend comprises between 70-98 parts cellulosic fluff pulp and between 2-30 parts polyolefin or polyester pulp or fiber and has a density between about 0.02 and about 0.50 g/cc,
characterized by dielectrically heating the resulting blend in order to bond the blend in less than 15 seconds without the aid of any dielectric sensitizing material.

2. The process according to claim 1 further comprising the step of adding up to about 30 parts of a super absorbent polymer to the resulting blend before it is dielectrically heated.

3. The process according to claim 1 wherein the polyolefin pulp or fiber comprises polyethylene.

4. The process according to claim 1 wherein the cellulosic fluff pulp comprises wood pulp or cotton linters.

5. The process according to claim 1 wherein the resulting blend is dielectrically heated by applying RF energy or microwave energy.

6. The process according to claim 5 wherein between 70 and 2500 MHz of RF energy are applied to dielectrically heat the resulting blend.

7. A dielectrically bonded article having a density between about 0.02 and about 0.50 g/cc comprising:
(a) 70-98 parts of a cellulosic fluff pulp;
(b) 2-30 parts of a fusible polyolefin or polyester pulp or fiber having a melting point less than 150°C; and
wherein the tensile strength of the interior portion of the bonded article is at least 120% greater than the tensile strength of any surface portion of the bonded article.

8. The article according to claim 7 wherein the article further comprises up to 30 parts of a super absorbent polymer.

9. The article according to claim 7 wherein the fusible polyolefin pulp or fiber comprises polyethylene.

10. The article according to claim 7 wherein the cellulosic fluff pulp comprises wood pulp or cotton linters.

11. The article according to claim 7 wherein the article comprises an absorptive core of a baby diaper, an adult incontinence product or a feminine pad.

12. The article according to claim 7 wherein the article comprises furniture padding, cushioning material or a polishing pad.

## Patentansprüche

1. Verfahren zum Verbinden einer Mischung von zellulosischer Faserflaumpulpe und schmelzbarer Polyolefin- oder Polyesterpulpe oder -faser, umfassend die Schritte des innigen Mischens trockener zellulosischer Faserflaumpulpe mit einer Polyolefin- oder Polyesterpulpe oder -faser mit einem Schmelzpunkt von weniger als 150°C, derart, daß die erhaltene Mischung zwischen 70 bis 98 Teile zellulosischer Faserflaumpulpe und zwischen 2 bis 30 Teilen Polyolefin- oder Polyesterpulpe oder -faser umfaßt und eine Dichte zwischen etwa 0,02 und etwa 0,50 g/cc aufweist,
gekennzeichnet durch dielektrisches Erhitzen der erhaltenen Mischung, um die Mischung in weniger als 15 Sekunden ohne die Zuhilfenahme irgendeines dielektrischen Sensibilisierungsmaterials zu verbinden.

2. Verfahren nach Anspruch 1, welches zusätzlich den Schritt des Zugebens von bis zu etwa 30 Teilen eines superabsorbierenden Polymeren zu der erhaltenen Mischung umfaßt, bevor sie dielektrisch erhitzt wird.

3. Verfahren nach Anspruch 1, bei welchem die Polyolefinpulpe oder -faser Polyethylen umfaßt.

4. Verfahren nach Anspruch 1, bei welchem die zellulosische Faserflaumpulpe Holzpulpe oder Baumwollinters umfaßt.

5. Verfahren nach Anspruch 1, bei welchem die erhaltene Mischung durch Anwendung von Radiofrequenzenergie oder Mikrowellenenergie dielektrisch erhitzt wird.

6. Verfahren nach Anspruch 5, bei welchem zwischen 70 und 2500 MHz Radiofrequenzenergie angewandt wird, um die erhaltene Mischung dielektrisch zu erhitzen.

7. Ein dielektrisch verbundener Gegenstand mit einer Dichte zwischen etwa 0,02 oder etwa 0,50 g/cc, umfassend:
(a) 70 bis 98 Teile einer zellulosischen Faserflaumpulpe;
(b) 2 bis 30 Teile einer schmelzbaren Polyolefin- oder Polyesterpulpe oder -faser mit einem Schmelzpunkt von weniger als 150°C; und
wobei die Zugfestigkeit des inneren Teiles des verbundenen Gegenstandes wenigstens 120 % größer ist als die Zugfestigkeit irgendeines Oberflächenteiles des verbundenen Gegenstandes.

8. Gegenstand nach Anspruch 7, wobei der Gegenstand darüber hinaus bis zu 30 Teile eines superabsorbierenden Polymeren umfaßt.

9. Gegenstand nach Anspruch 7, bei welchem die schmelzbare Polyolefinpulpe oder -faser Polyethylen umfaßt.

10. Gegenstand nach Anspruch 7, bei welchem die zellulosische Faserflaumpulpe Holzpulpe oder Baumwollinters umfaßt.

11. Gegenstand nach Anspruch 7, wobei der Gegenstand einen Absorptionskern einer Babywindel, eines Blasenschwächeproduktes für Erwachsene oder einer Monatsbinde umfaßt.

12. Gegenstand nach Anspruch 7, wobei der Gegenstand Möbelpolsterung, Polstermaterial oder ein Polierkissen umfaßt.

## Revendications

1. Un procédé de liaison d'un mélange de pâte de duvet cellulosique et de pâte ou fibre de polyoléfine ou polyester fusible comprenant les étapes de mélange intime d'une pâte de duvet cellulosique sèche avec une pâte ou fibre de polyoléfine ou polyester dont le point de fusion est inférieur à 150°C, de telle sorte que le mélange ainsi obtenu comprenne entre 70 et 98 parties de pâte de duvet cellulosique et entre 2 et 30 parties de pâte ou fibre de polyoléfine ou polyester et que sa densité soit comprise entre environ 0,02 et environ 0,50 g/cc, caractérisé en ce que l'on chauffe par voie électrique le mélange obtenu pour lier le mélange en moins de 15 secondes sans faire appel à l'aide d'aucun agent de sensibilisation diélectrique.

2. Procédé selon la revendication 1, comprenant en outre l'étape d'addition de jusqu'à environ 30 parties d'un polymère superabsorbant au mélange obtenu avant qu'il ne soit chauffé par voie diélectrique.

3. Procédé selon la revendication 1, dans lequel la pâte ou fibre de polyoléfine comprend du polyéthylène.

4. Procédé selon la revendication 1, dans lequel la pâte de duvet cellulosique comprend de la pâte de bois ou des déchets de coton.

5. Procédé selon la revendication 1, dans lequel le mélange obtenu est chauffé par voie diélectrique par application d'énergie RF ou d'énergie de micro-onde.

6. Procédé selon la revendication 5, dans lequel entre 70 et 2500 MHz d'énergie RF sont appliqués pour chauffer par voie diélectrique le mélange obtenu.

7. Un article lié par voie diélectrique présentant une densité entre environ 0,02 et environ 0,50 g/cc comprenant :
(a) 70 à 98 parties d'une pâte de duvet cellulosique;
(b) 2 à 30 parties d'une pâte ou fibre de polyoléfine ou polyester fusibles dont le point de fusion est inférieur à 150°C; et
dans lequel la résistance à la traction de la partie inférieure de l'article lié est d'au moins 120% supérieure à celle de la résistance à la traction d'une portion quelconque de la surface de l'article lié.

8. L'article selon la revendication 7, dans lequel l'article comprend encore jusqu'à 30 parties d'un polymère super absorbant.

9. L'article selon la revendication 7, dans lequel la pâte ou fibre fusibles de polyoléfine comprend du polyéthylène.

10. l'article selon la revendication 7, dans lequel la pâte de duvet cellulosique comprend de la pâte de bois ou des déchets de coton.

11. L'article selon la revendication 7, dans lequel l'article comprend un intérieur absorbant d'une couche pour bébé, d'un produit de protection contre l'incontinence des adultes ou une serviette hygiénique.

12. L'article selon la revendication 7, dans lequel l'article constitue un coussin d'ameublement, un matériau pour coussin ou un tampon de polissage.
